# EUROPEAN PATENT APPLICATION

(11) **EP 4 006 156 A1**
(43) Date of publication of application: **01.06.2022**
(21) Application number: 20847292.8
(22) Date of filing: 26.06.2020
(51) Int. Cl.: C12N 15/63, C12N 15/75, C12N 9/10, C12N 1/20, C12R 1/07

(54) **BACTERIA OF THE GENUS BACILLUS PRODUCING HYALURONIC ACID**

(30) Priority: 26.07.2019 RU 2019123612
(71) Applicant: Obshchestvo s Ogranichennoi Otvetstvennostyu "Centr Transfera Biotechnologiy Oka-Biotech", Moscow, 119421 (RU)
(72) Inventor: MIRONOV, Aleksandr Sergeevich, Moscow, 105094 (RU); ERRAIS, Lopes Lyubov, Moscow, 105094 (RU); KOROLKOVA, Natalya Valentinovna, Moscow, 113519 (RU); BAYEVA, Olga Viktorovna, Moscow, 119333 (RU); BATTALOVA, Irina Yunosovna, Tuchkovo, 143132 (RU); BROVKIN, Alexei Nikolaevich, Moscow, 117420 (RU); CMIRNOV, Petr Vladimirovich, Moscow, 117042 (RU); RYKOV, Sergey Viktorovich, Shkolnoe, 352621 (RU); URCHENKO, Uliay Valerievna, Moscow, 115597 (RU); KALUJSKIY, Vasiliy Evgenevich, Moscow, 119421 (RU)
(74) Representative: Osmans, Voldemars
(86) International application number: PCT/RU2020/000311
(87) International publication number: WO 2021/020995

(57) **Abstract**

The present invention relates to a transformed cell of bacteria of the genus *Bacillus,* more particularly *B. subtilis,* which produces hyaluronic acid, the cell comprising in its chromosome a fusion of heterologous *hasA* gene from *S. equisimilis* with *tuaD* gene of *B. subtilis* under control of the hybrid tandem promoter P_{rpsF-gsiB}, and optionally *gtaB* gene under control of the hybrid tandem promoter P_{rpsF-gsiB} and/or *gcaD-prs* operon under control of the hybrid tandem promoter P_{rpsF-gsiB}. The present invention also relates to a method for producing hyaluronic acid by said transformed cell.

## Description

### TECHNICAL FIELD

The present invention relates to biotechnology, in particular to a hyaluronic acid-producing bacterium of the genus *Bacillus*, more particularly, *Bacillus subtilis* bacterium, and a method for microbial synthesis of hyaluronic acid by culturing the bacterium.

### BACKGROUND ART

Discovery of functions and mechanisms of biological action of a variety of biopolymers helps to develop new products and preparations based thereon. One of such biopolymers of animal origin is hyaluronic acid. Hyaluronic acid, hyaluronate, or hyaluronan, (C₁₄H₂₁NO₁₁)ₙ, is an organic compound from the group of non-sulphated glycosaminoglycans. The presence of numerous sulphated groups in related glycosaminoglycans gives rise to a wide range of diverse isomers, which is not the case with hyaluronic acid, said acid being always chemically identical regardless of production methods and sources thereof. The hyaluronic acid molecule is built from repeated fragments of D-glucuronic acid and N-acetyl-D-glucosamine, linked by β-(1-3)-glycosidic bonds. Sugar fragments are based on a glucopyranose ring with various substituents (acetamide group, hydroxyl and carboxyl functional groups). The hyaluronic acid molecule is characterized by forming a lot of intramolecular hydrogen bonds both within the molecule and between adjacent carbohydrate residues located at a significant distance from one another, and, furthermore, between neighboring molecules through carboxylic and acetamide groups in aqueous solution. It is acidic due to presence of unprotonated carboxyl group. The acidic properties of hyaluronic acid allow for obtaining water-soluble salts with alkali metals. In the body, hyaluronic acid is found in the form of sodium hyaluronate salt. Hyaluronic acid is an anionic linear polysaccharide having various molecular weights. The molecular weight depends on production method, though all of the obtained hyaluronate is chemically identical to the standard one due to the absence of isomerism.

Many functions of hyaluronate have been discovered in the body. Hyaluronate is found in the hyaline cartilage, synovial articular fluid, and in skin tissue both in dermis and epidermis. Hyaluronate is also involved in many physiological functions, such as adhesion, development, cell motility, carcinogenesis, angiogenesis and wound healing. Due to its unique physical and biological properties, hyaluronate is often used in eye and joint surgery, and is being developed for use in other medical procedures. Hyaluronate preparations have also been developed for use in orthopedics, rheumatology and dermatology. In many cosmetic compositions, hyaluronate is included as an active ingredient.

All known methods for producing hyaluronic acid can be divided into two groups, i.e. the physicochemical method, which consists in extracting hyaluronate from mammalian or avian tissue material, for example, bovine vitreous bodies, chicken combs, or umbilical cords of newborns), and the microbial method based on bacterial producers.

Previously, a method of isolating hyaluronic acid from rooster combs was most widely used, because of the raw material availability and high content of the biopolymer therein. The extraction is carried out with a mixture of acetone-chloroform (for proteins removal), water, or a mixture of water and alcohol (propyl or tert-butyl alcohol) followed by adsorption on activated carbon, by using electrophoresis or an ion exchange resin. The analysis of animal sources of hyaluronate makes it evident that the supply of raw materials for industrial production of said polysaccharide is scarce and cannot completely satisfy the ever-growing demand for hyaluronate. In addition, isolation of hyaluronate from raw materials of animal origin is often complicated by the fact that in mammalian or avian tissues and organs this biopolymer is complexed with proteins; the presence of related glycosaminoglycans also hampers hyaluronate isolation. Using preparations of animal origin poses another potential risk consisting in their contamination with nucleic acids, prions and viruses.

A biotechnological method of making hyaluronate, based on culturing microorganisms - hyaluronate producers, is devoid of these disadvantages. The raw materials for production of polysaccharides by microbiological synthesis are represented by available compounds and components, namely: glucose, yeast extract, mineral salts, etc. Hyaluronate production based on microbiological synthesis does not depend on supply of animal raw materials, and is easily scaled up and redesigned in case of change in market conditions. International publications WO 99/51265 and WO 00/27437 disclose methods for producing hyaluronic acid by fermentation using a *Pasteurella multocida* strain. US patent No. 4,801,539 and International Publication WO99/23227 describe methods of fermentation for producing hyaluronic acid using strains of *Streptococcus zooepidemicus* and *Streptococcus equisimilis* with specified yields of about 3.6 g of hyaluronic acid per liter. European patents EP 0694616 and EP 2046969 describe methods of fermentation using an improved *Streptococcus zooepidemicus* strain with a specified yield of about 3.5 g of hyaluronic acid per liter.

Microorganisms used to produce hyaluronic acid by fermentation are strains of pathogenic bacteria, primarily various species of *Streptococcus.* Group A and group C streptococci of surround themselves with an antigenic capsule consisting of hyaluronate which is identical in composition to that found in connective tissues and joints.

Hyaluronan synthase is the last and key enzyme in the hyaluronic acid biosynthesis pathway. Hyaluronan synthases of vertebrates, bacterial pathogens, and algal viruses have been described (DeAngelis, P. L., 1999, Cell. Mol. Life Sci, 56: 670682). International publication WO 99/23227 describes Group I hyaluronate synthase of *Streptococcus equisimilis.* International publications WO 99/51265 and WO 00/27437 describe Group II hyaluronate synthase from *Pasteurella multocida.* A hyaluronan synthase operon of *Streptococcus pyogenes* has been described, said operon consisting of three genes, *hasA*, *hasB,* and *hasC*, encoding hyaluronate synthase, UDP glucose dehydrogenase, and UDP glucose pyrophosphorylase, respectively (Proc. Natl. Acad. Sci. USA. 98, 4658-4663, 2001). International publication WO 99/51265A describes a nucleic acid segment containing a hyaluronate synthase coding region of *Streptococcus equisimilis.*

Upon identification of the genes involved in hyaluronate biosynthesis, it became possible to carry out the genetic modification of a large group of bacteria (*Bacillus*, *Agrobacterium*, *E. coli* and *Lactococcus*) in order to use them as hyaluronate producers (WO 2012/032153, Appl. Microbiol. Biotechnol. 84:63-69, 2009).

Developed recombinant *B. subtilis* strains showed promising results in terms of the hyaluronate production (US Application publication 2014/0038235, and RU patent No. 2346049 of Novozymes company). The expression system used in these strains includes the heterologous *hasA* gene cloned from *S. equisimilis* and present in the single operon with one or more natural *B. subtilis* genes, i.e. homologues of *hasB*, *hasC,* and *hasD* genes of *Streptococcus.* The advantage of these producers is that the resulting product is free of streptococcal exotoxins and endotoxins.

Though the hyaluronate production was significantly improved through creating the above microbial strains, or due to improving the production process, it is necessary to develop more efficient processes for hyaluronate production to satisfy the growing demand for this compound for medical and veterinary applications, and cosmetology.

### SUMMARY OF INVENTION

The object of the group of inventions is development of more efficient method for microbial synthesis of hyaluronic acid. This object has been achieved by constructing a hyaluronic acid producing bacterium belonging to the genus *Bacillus*, in particular, *Bacillus subtilis* bacterium, comprising in its chromosome a fusion of heterologous *hasA* gene from *S. equisimilis* with *tuaD* gene of *B. subtilis* under control of a hybrid tandem PrpsF-gsiB promoter, and optionally comprising in its chromosome:
- *gtaB* gene under control of the hybrid tandem PrpsF-gsiB promoter; and/or
- *gcaD-prs* operon under control of the hybrid tandem PrpsF-gsiB promoter.

The above-described object has also been achieved by developing a method for microbial synthesis of hyaluronic acid by culturing said bacterium in a nutrient medium followed by isolating the hyaluronic acid from the culture broth.

The present invention relates to a hyaluronic acid producing bacterium of the genus *Bacillus,* in particular *B. subtilis* bacterium, comprising in its chromosome a fusion of the heterologous *hasA* gene from *S. equisimilis* with *tuaD* gene of *B. subtilis* under control of the hybrid tandem P_{rpsF-gsiB} promoter, and further optionally comprising in its chromosome:
- *gtaB* gene under control of the hybrid tandem PrpsF-gsiB promoter; and/or
- *gcaD-prs* operon under control of the hybrid tandem P_{rpsF-gsiB} promoter.

The present invention also relates to the above bacterium having in its chromosome the *gtaB* gene under control of the hybrid tandem PrpsF-gsiB promoter.

The present invention also relates to the above bacterium comprising in its chromosome the *gcaD-prs* operon under control of the hybrid tandem PrpsF-gsiB promoter.

The present invention also relates to the above bacterium comprising in its chromosome the *gtaB* gene under control of the hybrid tandem PrpsF-gsiB promoter, and the *gcaD-prs* operon under control of the hybrid tandem PrpsF-gsiB promoter.

The present invention also relates to the above bacterium which is *B. subtilis.*

The present invention also relates to a method for hyaluronic acid production, said method comprising:
(a) culturing the above bacterium in a nutrient medium suitable for hyaluronic acid production, and
(b) isolating the hyaluronic acid from the culture broth.

The present invention also relates to the method for hyaluronic acid production using the nutrient medium of the following composition (wt. %):

| |
|---|
| Sucrose - 8.0; |
| Maltose - 4.0; |
| Yeast extract - 0.5; |
| Urea - 0.6; |
| KH₂PO₄ - 0.6; |
| K₂HPO₄ - 1.4; |
| (NH₄)₂SO₄ - 0.3; |
| Sodium Citrate - 0.2; |
| Casamino acids - 0.04; |
| MgSO₄ - 0.3. |

### BRIEF DESCRIPTION OF DRAWINGS

Figure 1 schematically show a representation of hyaluronic acid biosynthesis. Genes and hyaluronic acid biosynthesis reactions regulated by said genes are shown.
Figure 2 shows the nucleotide sequence of the hybrid P_{rpsF-gsiB} promoter. Bold capital letters denote -35 and -10 promoter regions; the ribosome binding site is underlined. Transcription start sites are designated as +1. The junction of the P_{rpsF} and P_{gsiB} promoters is indicated by an arrow.
Figure 3 shows the structure of pDG268 plasmid.
Figure 4 shows efficiency of the hybrid P_{rpsF-gsiB} promoter versus single P_{rpsF} promoter during fermentation of *B. subtilis* strain with the exemplary reporter *lacZ-*gene encoding β-galactosidase.
Figure 5 shows the structure of pLE1 vector.
Figure 6 shows the relative transcription levels for the *hasA*, *tuaD*, *gtaB,* and *gcaD* genes in the AM2640, AM2681, AM2686, and AM2694 strains.

### DESCRIPTION OF EMBODIMENTS

The term "bacterium of the genus *Bacillus*" means a bacterium that belongs to the genus *Bacillus* according to classification known to those skilled in the field of microbiology. In particular, a bacterium selected from the group including *Bacillus alkalophilus*, *Bacillus amyloliquefaciens*, *Bacillus brevis*, *Bacillus circulans*, *Bacillus clausii*, *Bacillus coagulans*, *Bacillus firmus*, *Bacillus lautus*, *Bacillus lentus*, *Bacillus licheniformis*, *Bacillus megaterium*, *Bacillus pumilus*, *Bacillus stearothermophilus*, *Bacillus subtilis,* and *Bacillus thuringiensis,* is a bacterium of the genus *Bacillus.*

The genus *Bacillus* is known to include over 200 bacterial species, and the most practically important of them not only share common features with regard to morphology, physiology and cell culture, but also show certain similarities between their genome organization and gene expression mechanisms. Direct experiments have shown that, for example, the promoter of *aprE* extracellular proteinase gene of *B. subtilis* is able to initiate synthesis of a reporter protein in the cells of various bacilli species, sometimes even to a greater extent compared to *B. subtilis* cells (Genome Biology, 5, 77, 2004).

The term "bacterium of the genus *Bacillus*" means a bacterium that belongs to the *Bacillus subtilis* species according to the classification known to those skilled in the art of microbiology.

The term "hyaluronic acid" as used herein means a non-sulfated glucosaminoglycan composed of repeating disaccharide units consisting of N-acetylglucosamine (GlcNAc) and glucuronic acid (GlcUA), linked together with alternating β-1,4- and β-1,3-glycosidic bonds. Hyaluronic acid sodium salt is also known as hyaluronan, or hyaluronate.

The term "hyaluronan synthase" as used herein refers to a synthase (EC 2.4.1.212) that catalyzes elongation of the hyaluronan chain by the addition of saccharide precursors GlcUA and GlcNAc. In the present invention, a fusion of heterologous *hasA* gene from *S*. *equisimilis* with the *tuaD* gene of *B. subtilis* under control of the hybrid tandem PrpsF-gsiB promoter is used for producing hyaluronic acid by the *Bacillus* bacterial cells, in particular, *B. subtilis.*

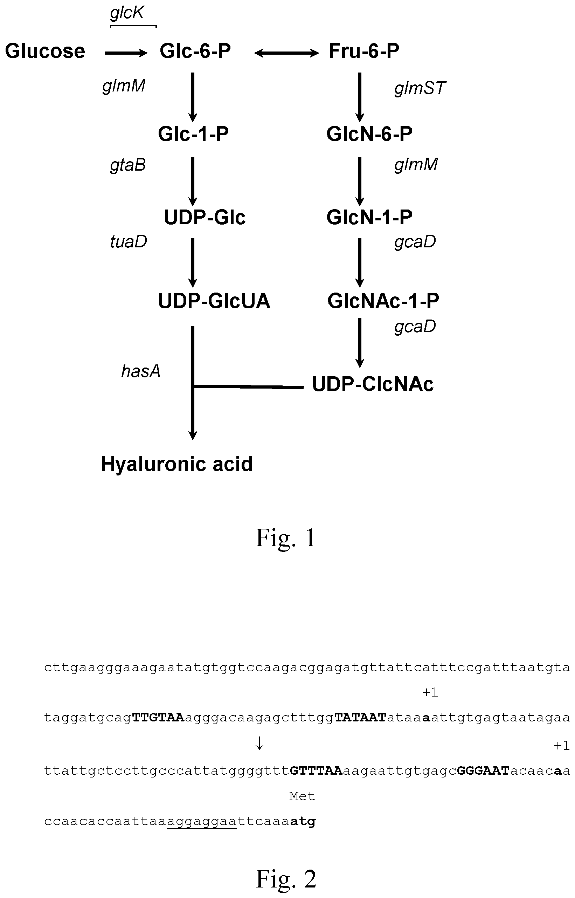

Genes involved in the biosynthesis of hyaluronic acid saccharide precursors include *tuaD* gene of *B. subtilis* encoding UDP-glucose-6-dehydrogenase (EC 1.1.1.22), *gtaB* gene of *B. subtilis* encoding UDP-glucosopyrophosphorylase (EC 2.7.7.9), *gcaD* gene of *B. subtilis* encoding UDP-N-acetylglucosamine pyrophosphorylase (EC 2.7.7.23), and *prs* gene encoding phosphoribosyl pyrophosphate synthase (EC 2.7.6.1) (see Figure 1).

The term "heterologous gene" means that the gene is isolated from a chromosome of an organism other than the bacterium of the genus *Bacillus,* in particular, *B. subtilis.* The bacterium comprising the heterologous *hasA* gene from *S. equisimilis* in its chromosome is obtained using standard recombinant DNA techniques, in particular, transformation and transfection.

In addition to the heterologous *hasA* gene from *Streptococcus equisimilis*, the host *Bacillus* cell, in particular, *B. subtilis* cell can further contain one or more second nucleic acid constructs carrying one or more genes that encode enzymes for biosynthesis of the saccharide precursor under control of the hybrid tandem PrpsF-gsiB promoter.

The terms "tandem PrpsF-gsiB promoter is functionally linked" to the target gene or "the gene is under control of the hybrid tandem PrpsF-gsiB promoter" mean that the native promoters of these genes have been replaced with the P_{rpsF-gsiB} promoter. The tandem P_{rpsF-gsiB} promoter is a fusion of the *rpsF* gene promoter, which encodes the synthesis of the ribosomal S6 protein, and the *gsiB* gene promoter responsible for synthesis of the general stress protein (PgsiB). The P_{rpsF} promoter contains the -35 region close to the consensus sequence (TTGTAA), and the -10 consensus sequence region (TATAAT). The P_{rpsF} promoter is one of the strongest promoters in the *B. subtilis* genome; it provides the maximum transcription level in the logarithmic phase of bacterial growth. Transcription initiation at this promoter involves the RNA polymerase vegetative sigma subunit (σ^{A}). The nucleotide sequence of *rpsF* gene of *B. subtilis* is available in GenBank under accession number GeneID: 937919 (nucleotide positions 4199445 to 4199732 of NC_000964.3). Initiation of the *gsiB* gene transcription involves an alternative sigma subunit σ^{B}, and the transcription level reaches its maximum during the stationary phase of bacterial growth. The nucleotide sequence of *gsiB* gene of *B. subtilis* is available in GenBank under accession number GeneID: 938235 (nucleotide positions 494506 to 494877 of NC_000964.3).

The nucleotide sequence of the tandem P_{rpsF-gsiB} promoter used in the present invention is shown on Figure 2 and set forth in SEQ ID NO: 1 of the Sequence Listing.

The tandem P_{rpsF-gsiB} promoter can be obtained by any method known to a person skilled in the art, in particular, chemical synthesis, polymerase chain reaction with overlapping primers (overlap extension PCR), P*rpsF*:P*gsiB* fusion, and the like.

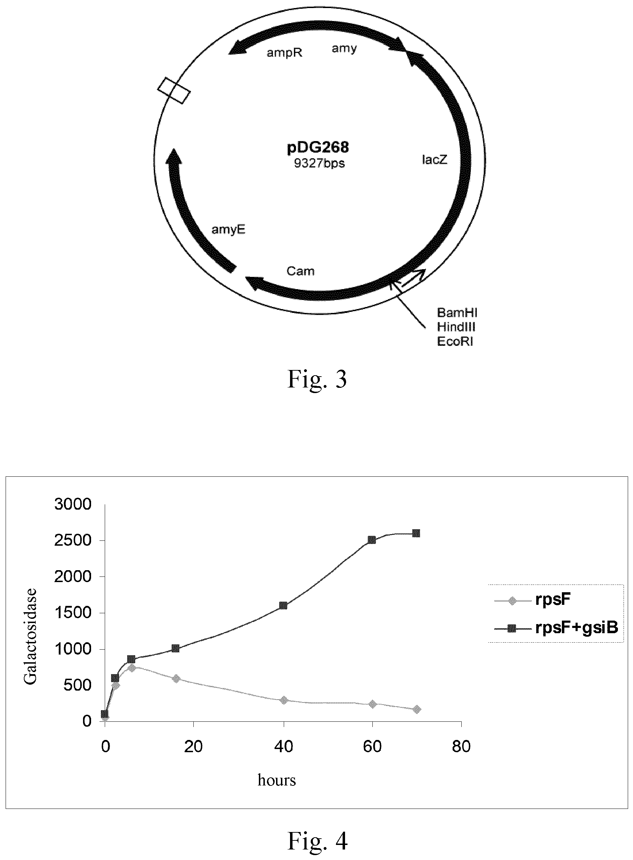

Authors of the present invention have shown that use of the hybrid P_{rpsF-gsiB} promoter allows one to obtain an optimal expression profile of a target gene during bacterial culturing, and is very promising for enhancing expression of the target genes used to obtain target products, in particular, hyaluronic acid. For example, activity of the hybrid P_{rpsF-gsiB} promoter increases during fermentation and reaches the maximum level in the late stationary growth phase. The activity profile of the hybrid P_{rpsF-gsiB} promoter, along with the performance of the hybrid P_{rpsF-gsiB} promoter compared to the single P_{rpsF} promoter during fermentation of *B. subtilis* strains are exemplified on Figure 4 using reporter *lacZ* gene encoding β-galactosidase.

In the present invention, a number of constructs producing hyaluronic acid, namely AM2640, AM2681, AM2686, and AM2694, was obtained based on the original *B. subtilis* 168 strain. More detailed description of these genetic constructs and methods for constructing thereof is given in the following examples.

### Characteristics of Genetic Constructs

The obtained *B. subtilis*-based genetic constructs are superproducers of the hyaluronic acid and have the following properties.

### Cultural and Morphological Traits

A gram-positive spore-forming rod with rounded ends and marginal spores. On day 2, after plating onto LB agar (Miller, J. H., 1979. Experiments in Molecular Genetics, Mir, Moscow) and culturing at 37°C, colonies with uneven edges of 3 to 4 mm in diameter are observed, while culturing in the minimal Spizeisen medium (J. Bacteriol. 81:741-746, 1961) results in formation of similar colonies of 2 to 3 mm in diameter.

### Physiological and Biochemical Traits

Able to ferment saccharides such as glucose, lactose, galactose, fructose, arabinose, maltose, xylose, trehalose, and starch hydrolysate, as well as alcohols such as glycerol, mannitol, and sorbitol, organic acids such as gluconic, fumaric, citric, and succinic acids, and similar compounds.

### Storage

Lyophilized, or in liquid nitrogen vapors.

### Stability

The obtained *B. subtilis*-based genetic constructs are stable and do not lose ability to synthesize hyaluronic acid after 10 passages on a complete medium.

### Method of the present invention

Methods of the present invention include a method for producing hyaluronic acid, wherein the method comprises stages of culturing the bacterium of the present invention in a nutrient medium in order to produce and accumulate the target product in the nutrient medium, followed by isolation of the target product from the culture broth.

According to the present invention, the cultivation, collection and purification of the target product from the culture broth or the equivalent of the culture broth can be carried out by a method similar to conventional cultivation methods, where a target product is produced by a microorganism. The nutrient medium used for cultivation can be either synthetic or natural, provided that the medium comprises carbon and nitrogen sources, as well as mineral supplements, and, if necessary, appropriate amounts of nutritional supplement required for microbial growth. Carbon sources include various carbohydrates such as glucose and sucrose, various organic acids, or alcohols such as ethanol and glycerin. Various ammonium salts such as ammonium sulfate, or other nitrogenous compounds such as ammonia and amines, natural nitrogen sources such as peptone and microbial enzymatic lysates can be used as nitrogen sources. Potassium monophosphate, magnesium sulfate, sodium chloride, ferrous sulfate, manganese sulfate, calcium chloride and similar compounds can be used as mineral supplements.

The cultivation is carried out under aerobic conditions while mixing (for instance, with shaking, stirring, bubbling, etc.) at temperatures of 30 to 38 °C, preferably at 37 °C. The pH is maintained in the range of 5 to 9, preferably 6.5-7.2, and is adjusted by addition of buffer solutions, or ammonia, calcium carbonate, various acids or bases. Cultivation for 1 to 3 days leads to accumalation of the target product, hyaluronic acid, in the culture broth. The method allows to accumulate hyaluronic acid in both the culture growing process and stationary growth phase after the biomass scale-up is completed.

When cultivation is over, solid residues such as cells can be removed from the culture broth by centrifugation or membrane filtration; then, hyaluronic acid can be collected and purified by stepwise precipitation with cetylpyridinium and isopropyl alcohol followed by the column chromatography purification on adsorption and ion-exchange carriers. Ultrafiltration and lyophilization can also be used at final polishing steps.

The hyaluronic acid concentration in the cell-free culture broth is measured by carbazole assay (T. Bitter, H.M. Muir. Analytical Biochemistry, v.4, p. 330-334, 1962).

The present invention will be described in more detail with reference to the examples.

The following examples are provided to clarify the essence of the present invention, and are not intended to limit the scope of the present invention defined by the appended claims.

### EXAMPLES

### Example 1. Construction of pDG268-P plasmid comprising the hybrid P_{rpsF-gsiB} promoter.

The present invention provides an engineered tandem promoter functional in bacteria of genus *Bacillus*, in particular, *B. subtilis*, said tandem promoter comprises the nucleotide sequences of the *rpsF* gene promoter and the *gsiB* gene promoter, wherein the *rpsF* gene promoter is located upstream of the *gsiB* gene promoter. Preparation of this promoter is disclosed in a pending patent application entitled "Tandem promoter functional in bacteria of the genus *Bacillus*, transformed *Bacillus* bacterium producing useful metabolite containing said promoter, and method for producing target product using said bacterium". The P_{rpsF} promoter is one of the strongest promoters in the *B. subtilis* genome; it provides the maximal transcription level in the logarithmic phase of bacterial growth. Transcription initiation at this promoter involves the RNA polymerase vegetative sigma subunit (σ^{A}). Transcription of the *gsiB* gene responsible for synthesis of the general stress protein is initiated with the involvement of the alternative sigma subunit σ^{B}, and the transcription level reaches its maximum during the stationary phase of bacterial growth. The tandem promoter comprises: -35 and -10 regions and the transcription start sites of the *rpsF* gene promoter, -35 and -10 regions and the transcription start sites of the *gsiB* gene promoter, and a ribosome-binding site (Figure 2).

The hybrid P_{rpsF-gsiB} promoter shown in Fig. 2, having the nucleotide sequence as set forth in SEQ ID NO: 1 of the Sequence Listing, was treated with *Eco*RI and *Bam*HI restriction enzymes, and cloned into the corresponding sites of pDG268 plasmid (Cell 111: 747756, 2002), which includes a cassette containing a cloning polylinker, a reporter *lacZ* gene without its native promoter and the chloramphenicol resistance gene (Cm^{R}) (see Fig. 3). The cassette was flanked with the *amyE* gene fragments, thus allowing to integrate the vector with the cloned fragment into the *amyE* locus on the chromosome of the *Bacillus* genus bacterium, in particular, *B. subtilis.* The resulting plasmid named pDG268-P was used to transform competent *E. coli* TG1 strain cells. The transformants were selected on LB medium containing 100 µg/mL ampicillin. Plasmid DNA was purified on agarose gel and purified using columns (GFX PCR DNA and Gel Band Purification Kit from Illustra), or Bead DNA Gel Extraction Kit. The nucleotide sequence of the inserted DNA was confirmed by DNA sequencing with primer 1 (SEQ ID NO: 2, forward) and primer 2 (SEQ ID NO:3, reverse).

The resulting plasmid named pDG268-P, which comprises the hybrid P_{rpsF-gsiB} promoter, can be integrated into the *amyE* chromosome locus of the bacterium of the genus *Bacillus,* in particular, *B. subtilis.* Accordingly, prior to use for *B. subtilis* cells transformation, pDG268-P plasmid DNA was linearized using the *Xba*I restriction endonuclease to allow integration into the chromosome by double crossover instead of simple crossover. The competent cells of *B. subtilis* strain 168 were transformed with the pDG268-P plasmid DNA pretreated by *Xba*I restriction endonuclease, and transformants were selected on LB medium containing 10 µg/mL chloramphenicol. The plasmid integration into the *amyE* chromosomal locus was confirmed using indicator medium comprising 0.2% amylopectin.

Thus, the performed manipulations yielded the plasmid pDG268-P containing the hybrid P_{rpsF-gsiB} promoter and the adjacent reporter *lacZ* gene which allows to test activity of said hybrid promoter under fermentation conditions.

As is seen from Figure 2, the hybrid promoter contains two -35 and two -10 regions, in addition to the two transcription start sites, which, as assumed by the authors of the present invention without intending to be limited by any theory, can be used alternately at different stages of culture growth and provide optimal expression of genes controlled by said promoter (Figure 4).

### Example 2. Preparation of AM2640 genetic construct by introducing a fusion of hasA gene from S. equisimilis and tuaD gene of B. subtilis under control of the hybrid tandem PrpsF-gsiB promoter into chromosome of recipient Bacillus subtilis strain 168.

Construction of fusion of *hasA* gene from *S. equisimilis* and *tuaD* gene of *B. subtilis* under control of the hybrid tandem PrpsF-gsiB promoter was carried out in several steps. First, the *hasA* gene was chemically synthesized based on the nucleotide sequence of *hasA* gene from *S. equisimilis* and cloned into pUC57 vector. The resulting plasmid DNA was used for PCR amplification of the *hasA* gene of 1250 bp utilizing primer 3 (SEQ ID NO: 4) comprising *Bam*HI restriction endonuclease recognition site, and primer 4 (SEQ ID NO: 5) comprising 5'-terminal sequence complementary to the N-terminal region of the *tuaD* gene of *B. subtilis.*

PCR amplification was carried out in a 50 µL incubation mixture containing 3 ng of pUC57 plasmid DNA as a template, 20 pmol of each of primers 3 (SEQ ID NO: 4) and 4 (SEQ ID NO: 5), 1x PCR buffer, a dNTPs mixture (0.5 mM each), and 2 U of a thermostable *Taq* polymerase. The reaction was carried out in My Cycler BioRad thermal cycler programmed for 1 cycle of 95 °C for 5 min; 30 cycles of 95 °C for 30 s, 58 °C for 30 s, and 72 °C for 2 min each; and 1 cycle of 72 °C for 5 min. The products were analyzed by gel electrophoresis on 1.2% agarose gel in 1×TAE. The expected fragment was about 1,270 bp in size.

At the next step, the *tuaD* gene was PCR-amplified from *B. subtilis* chromosomal DNA using primer 5 (SEQ ID NO: 6) comprising a 5'-terminal sequence complementary to the C-terminus of *hasA* gene from *S. equisimilis* and primer 6 (SEQ ID NO: 7) comprising a *Not*I restriction endonuclease recognition site.

PCR amplification was carried out in a 50-µL incubation mixture containing 5 ng of *B. subtilis* chromosomal DNA as a template, 20 pmol of each of primers 5 (SEQ IDNO: 6) and 6 (SEQ ID NO: 7), 1× PCR buffer, a dNTPs mixture (0.5 mM each), and 2 U of a thermostable *Taq* polymerase. The reaction was carried out in My Cycler BioRad thermal cycler programmed for 1 cycle of 95 °C for 5 min; 30 cycles of 95 °C for 30 s, 58 °C for 30 s, and 72 °C for 2 min each; and 1 cycle of 72 °C for 5 min. The products were analyzed by electrophoresis on 1.2% agarose gel in 1×TAE. The expected fragment was about 1,450 bp in size.

Then the DNA fragments obtained using primer pairs 3/4 and 5/6 were aligned by annealing to each other by PCR using the following mode: PCR amplification in a 50 µL incubation mixture comprising 5 ng of each DNA fragment prepared using primer pairs 3/4 and 5/6, 1× PCR buffer, a dNTPs mixture (0.5 mM each), and 2 U of a thermostable *Taq* polymerase. The reactions were carried out in My Cycler BioRad thermal cycler programmed for 1 cycle of 95 °C for 5 min; 10 cycles of 95°C for 30 s, 52°C for 30 s and 72 °C for 2 min each, and 1 cycle of 72 °C for 5 min. Next, the following primers were added to the reaction mixture: primer 3 (SEQ ID NO: 4), which comprised a *Bam*HI restriction endonuclease recognition site, and primer 6 (SEQ ID NO: 7), comprising a *Not*I restriction endonuclease restriction site. PCR was carried out in the following mode: 25 cycles of 95 °C for 30 s each, 56 °C for 30 s, and 72 °C for 2.5 min; and 1 cycle of 72 °C for 5 min. The products were analyzed by electrophoresis on 1.2% agarose gel in 1×TAE. The expected fragment was about 2,700 bp in size.

The resulting 2,700 bp DNA fragment was treated with the *Bam*HI and *Not*I restriction endonucleases, and then cloned in the corresponding sites of the previously prepared pDG268-P plasmid comprising the hybrid tandem P_{rpsF-gsiB} promoter. The resulting plasmid, named pDG268-PHT, was used to transform competent *E. coli* TG1 cells. The transformants were selected on LB medium comprising 100 µg/mL ampicillin. Plasmid DNA was purified on agarose gel and eluted using columns (GFX PCR DNA and Gel Band Purification Kit, IIlustra) or DNA Gel Extraction Kit beads (from Fermentas). The nucleotide sequence of the DNA insert was confirmed by DNA sequencing using forward primer 1 (SEQID NO: 2) and reverse primer 2 (SEQ ID NO: 3).

At the final step, the produced pDG268-PHT plasmid was integrated into the *amyE* chromosomal locus of *B. subtilis.* Towards this end, pDG268-PHT plasmid DNA was linearized prior to transformation using the *Xba*I restriction endonuclease to ensure integration into the chromosome by double crossover instead of simple crossover. The competent cells *of B. subtilis* strain 168 were transformed with the pDG268P plasmid DNA pretreated with *Xba*I*,* and the transformants were selected on LB medium with 10 µg/mL chloramphenicol. The plasmid integration into the *amyE* chromosomal locus was confirmed using an indicator medium comprising 0.2% amylopectin.

Thus, the performed manipulations yielded a *B. subtilis*-based genetic construct comprising the fusion of *hasA* gene from *S. equisimilis* and *tuaD* gene of *B. subtilis* under control of the hybrid tandem PrpsF-gsiB promoter.

### Example 3. Preparation of AM2681 genetic construct by introducing gtaB gene under control of the hybrid tandem PrpsF-gsiB promoter into chromosome of the recipient Bacillus subtilis AM2640.

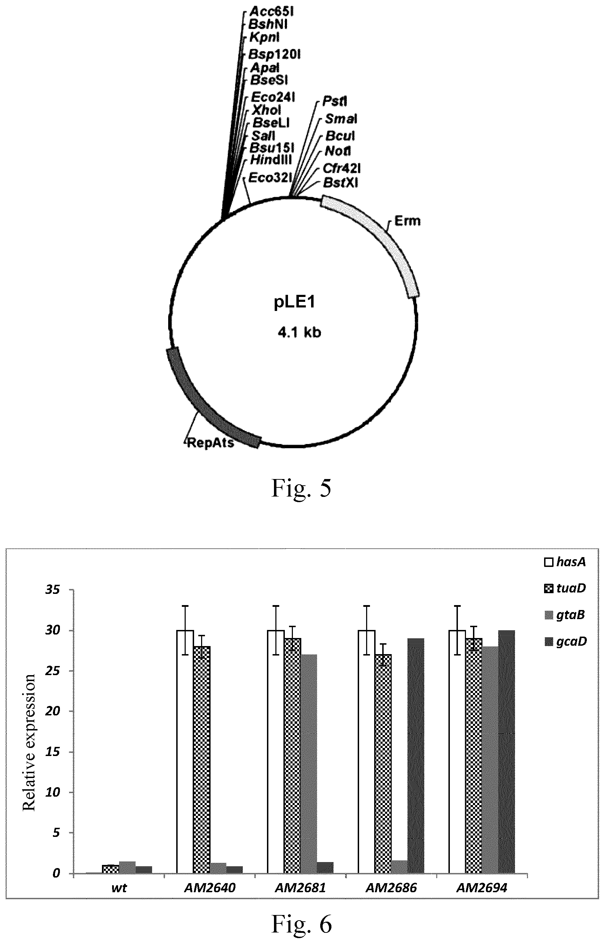

The *gtaB* gene in the resulting *B. subtilis* AM2640 construct was placed under control of the hybrid tandem PrpsF-gsiB promoter to enhance the gene expression. To this end, the P_{rpsF-gsiB} promoter region was cloned into pLE1 expression vector able to replicate in both bacteria of the genus *Bacillus* and *E. coli,* and comprising the erythromycin resistance gene (Figure 5). pLE1 vector is a deletion mutant version of pKS1 plasmid (FEMS Microbiol. Letters, 245: 315-319, 2005), wherein the kanamycin resistance marker has been deleted.

For this purpose, PCR amplification was carried out from pDG268-P plasmid DNA comprising the hybrid P_{rps-FgsiB} promoter in the presence of the tandem promoter flanking primer 7 (SEQ ID NO: 8) comprising a *Pst*I restriction site and primer 8 (SEQ IDNO: 9) comprising a *Hind*III restriction site.

The obtained 232 bp PCR product was purified on agarose gel, treated with *Pst*I and *Hind*III restriction endonucleases and cloned into pLE1 vector at the *Pst*I and *Hind*III sites. The obtained plasmid comprising the hybrid tandem P_{rpsF-gsiB} promoter was named as pLE3.

At the next stage, the promoter-proximal region of the *gtaB* gene was PCR-amplified from the *B. subtilis168* trpC2 strain chromosomal DNA using primer 9 (SEQ ID NO: 10), which comprised a *Sac*II site, and primer 10 (SEQ ID NO: 11) comprising a *Pst*I site.

The resulting 394 bp PCR product was purified on agarose gel, treated with the *Sac*II and *Pst*I restriction endonucleases and cloned into the *Sac*II and *Pst*I sites of the pLE3 vector comprising the P_{rpsF-gsiB} promoter. Next, the *gtaB* gene reading frame fragment was PCR- amplified from chromosomal DNA of the *B. subtilis*168 strain trpC2 using primer 11 (SEQ ID NO: 12) comprising a *Hind*III restriction endonuclease site, and primer 12 (SEQID NO: 13) comprising a *Kpn*I site.

The resulting 397 bp PCR product was purified on agarose gel, treated with the *Hind*III and *Kpn*I restriction endonucleases and cloned into the same sites of the pLE3 vector prepared on the previous stage and comprising the P_{rpsF-gsiB} promoter and the *gtaB* gene promoter-proximal region. The pLE5 gtaB'-PrpsF-gsiB-'gtaB plasmid construct, resulting from these manipulations, has the native promoter replaced with the P_{rpsF-gsiB} promoter.

Thus, the constructed pLE5 plasmid was used to transform the *E. coli* strain TG1, and the transformants were selected on the erythromycin-containing (300 µg/mL) medium at 30 °C. The resulting clones were PCR checked for presence of the pLE5 plasmid having the gtaB'-PrpsF-gsiB-'gtaB fragment cloned into it. The pLE5 (gtaB'-PrpsF-gsiB'gtaB) plasmid was isolated from the tested clones and used to transform the *B. subtilis* strain AM2640. The Em^{R} transformants were selected on the erythromycin-containing (3 µg/mL) medium. Several obtained Em^{R} clones were cultured overnight in the erythromycin-containing (3 µg/mL) liquid LB medium at 37 °C, and then plated on Petri dishes with the erythromycin-containing (3 µg/mL) LB medium and incubated at 37 °C for 24 h. The resulting Em^{R} recombinants were formed as a result of the pLE5 (gtaB'-PrpsF-gsiB'gtaB) plasmid integration into the corresponding chromosomal *gtaB* locus, confirmed by producing a 901-bp PCR fragment using primer 9 (SEQ ID NO: 10) and primer 12 (SEQ ID NO: 13). Several of the obtained Em^{R} clones were inoculated into liquid LB medium with no antibiotic and incubated on a rocking shaker at 30 °C for 48 h, and then the cultures were seeded in Petri dishes with LB medium containing no antibiotic and incubated at 30 °C for 24 h.

Finally, at the last step the grown clones were checked for the excision of the integrated pLE5 plasmid, which was assessed by the appearance of erythromycin-sensitive clones. The plasmid excision occurs either with maintenance of the wild type *gtaB* gene promoter in the chromosome, or with replacement of of the wild type promoter with the P_{rpsF-gsiB} promoter. Integration of the P_{rpsF-gsiB} promoter into the *B. subtilis* strain chromosome upstream of the *gtaB* gene was confirmed by producing a 791-bp PCR fragment using primer 1 (SEQ ID NO: 2) and primer 13 (SEQ ID NO: 14) with subsequent sequencing of the fragment. One of the *B. subtilis* AM2640 variants comprising the *gtaB* gene under control of the P_{rpsF-gsiB} promoter, named AM2681, accumulated up to 2.21 g/L hyaluronic acid during 48 h fermentation (Table 1).

### Example 4. Preparation of AM2686 and AM2694 genetic constructs by introducing the gcaD-prs operon under control of the hybrid tandem PrpsF-gsiB promoter into the chromosomes of Bacillus subtilis AM2640 and AM2681 recipients, respectively.

The gcaD-prs operon in the genome of the above-mentioned *B. subtilis* AM2640 genetic construct was placed under control of the hybrid tandem PrpsF-gsiB promoter to enhance its expression. Accordingly, at the first step, the promoter-proximal region of the gcaD-prs operon was PCR-amplified from *B. subtilis* 168 trpC2 DNA using primer 14 (SEQ ID NO: 15) comprising *Sac*II site and primer 15 (SEQ ID NO: 16) comprising *Pst*I site.

The obtained 419 bp PCR product was purified on agarose gel, treated with the *Sac*II and *Pst*I restriction endonucleases and cloned into *Sac*II and *Pst*I sites of pLE3 vector prepared on the previous stage and comprising the P_{rpsFgsiB} promoter.

Next, a fragment of the *gtaC* gene reading frame was PCR-amplified from *B. subtilis* 168 trpC2 chromosomal DNA using primer 16 (SEQ ID NO: 17) comprising a *Cla*I site and primer 17 (SEQ ID NO: 18) comprising *a KpnI* site.

The obtained 394 bp PCR fragment was purified on agarose gel, treated with the *Cla*I and *Kpn*I restriction endonucleases and cloned into the corresponding sites of the previously prepared pLE3 vector comprising the P_{rpsF-gsiB} promoter and the gcaD-prs operon promoter-proximal region. The pLE8 gcaD'-PrpsF-gsiB-'gcaD plasmid construct, resulting from these manipulations, has the native gcaD-prs promoter replaced with the P_{rpsF-gsiB} promoter.

Thus the obtained pLE8 plasmid was used to transform the *E. coli* strain TG1, and the Em^{R} transformants were then selected on the erythromycin-containing (300 µg/mL) medium at 30 °C. The resulting clones were PCR-checked for the presence of the pLE8 plasmid having the cloned gcaD'-PrpsF-gsiB-'gcaD fragment. The pLE8 (gcaD'-PrpsF-gsiB'gcaD) plasmids isolated from the tested clones were used to transform the previously prepared *B. subtilis* AM2640 and AM2681 constructs, and the Em^{R} transformants were selected on the erythromycin-containing (3 µg/mL) medium. Several Em^{R} clones resulting from transformation of AM2640 and AM2681 genetic constructs were cultured overnight in liquid LB medium containing erythromycin (3 µg/mL), and then plated on Petri dishes with LB medium containing erythromycin (3 µg/mL) and incubated for 24 h at 37 °C. The obtained Em^{R} recombinants were formed as a result of the pLE8 (gcaD'-PrpsF-gsiB-'gcaD) plasmid integration into the corresponding chromosomal *gcaD* locus, which was confirmed by producing a 891 bp PCR fragment using primer 14 (SEQ ID NO: 15) and primer 17 (SEQ ID NO: 18). Several obtained Em^{R} clones were inoculated into liquid LB medium with no antibiotic and incubated on a rocking shaker at 30 °C for 48 h, and then plated on Petri dishes with LB medium containing no antibiotic and incubated at 30 °C for 24 h.

Finally, on the last step, the grown clones were checked for excision of the integrated pLE8 plasmid, which was assessed by appearance of erythromycin-sensitive clones. The plasmid excision occurs either with maintenance of the wild type gcaD-prs operon promoter in the chromosome, or with replacement of the wild type promoter with the P_{rpsF-gsiB} promoter. The P_{rpsF-gsiB} promoter integration upstream of the gcaD-prs operon of the *B. subtilis* chromosome was confirmed by producing a 772 bp PCR fragment using primer 1 (SEQ ID NO: 2) and primer 18 (SEQ ID NO: 19), with subsequent sequencing of the fragment. According to one of the embodiments, the *B. subtilis* AM2640 and AM2681 genetic constructs comprising the gcaD-prs operon under control of the P_{rpsF-gsiB}promoter, named AM2686 and AM2694, accumulated up to 2.83 g/L and 3.21 g/L hyaluronic acid, respectively, during 48-h fermentation (Table 1).

### Example 5. Determination of target genes transcription levels in the produced genetic constructs by real-time PCR.

Transcription levels of the *hasA*, *tuaD*, *gtaB,* and *gcaD* genes were assessed by real-time PCR. To this end, cell cultures with the genetic constructs to be analyzed were grown to OD₆₀₀ = 1.4-1.6, and total RNA was isolated. The RNA was purified using RNeasy Mini Kit (Qiagen) according to the manufacturer's instructions. The obtained RNA preparations were treated with DNase I. The quality of the obtained total RNA was assayed on 1% agarose gel with the addition of formamide. The RNA amount was measured on a spectrophotometer at absorbance wavelength 260 nm. Prior to the reverse transcription reaction, RNA samples were treated with DNAse I (Thermo). The reverse transcription reaction was carried out in the presence of oligonucleotides specific for the genes of interest using the RT-PCR SuperScript III First-Strand Synthesis Kit (Invitrogen). Next, a 1 µl aliquot of the entire reverse transcription reaction volume was used as a template for PCR with real-time detection. The *rrnA* and *gyrB* genes expression levels were used for normalization.

The analysis was performed using a real-time PCR reagent kit (Sintol, Russia). The DTlite device (DNK-Tekhnologiya) was used for the amplification. The reaction products were analyzed by electrophoresis in 2% agarose gel to confirm expected sizes. Each of the reactions was performed in triplicate; results are expressed as averages. The transcription level was determined based on the threshold cycle values, given that the initial specific DNA fragments concentration increases as 2^{N}, where N is the number of cycles. The results of the transcription level analysis for the genes *hasA*, *tuaD*, *gtaB,* and *gcaD* in the AM2640, AM2681, AM2686 and AM2694 strains are shown in Figure 6.

As it is seen from the data on Figure 6, placing *hasA*, *tuaD*, *gtaB,* and *gcaD* genes under control of the tandem hybrid P_{rpsF-gsiB} promoter provides nearly equal almost 30-fold increase in their transcription levels.

### Example 6. Hyaluronic acid production by the engineered genetic constructs.

Culture inoculum of the claimed hyaluronic acid-producing *B. subtilis* genetic construct was obtained by culturing with shaking (200-220 rpm) for 18 h at 37 °C on LB medium of the following composition (wt. %):

| |
|---|
| Casein peptone - 1.0; |
| Yeast extract - 0.5; |
| NaCl - 0.5 |
| Glucose - 1.0; |
| MgSO₄ - 0.02. |

The obtained culture was inoculated into a fermentation medium of the following composition (wt. %):

| |
|---|
| Sucrose - 8.0; |
| Maltose - 4.0; |
| Yeast extract - 0.5; |
| Urea - 0.6; |
| KH₂PO₄ - 0.6; |
| K₂HPO₄ - 1.4; |
| (NH₄)₂SO₄ - 0.3; |
| Sodium citrate - 0.2; |
| Casamino acids - 0.04; |
| MgSO₄ - 0.3. |

Fermentation with the producer strain was performed for 48 h at 37 °C. Isolation of the hyaluronic acid from the culture liquid includes the following steps:

Removing the cell mass from the culture broth where the constructs were grown by centrifugation at 8,000 rpm. The pellet was discarded, and the supernatant was collected for future use.

Precipitating the hyaluronic acid with cetylpyridinium and isopropanol solution.

10% aqueous cetylpyridinium solution was added to the supernatant to a final concentration of 1%. The solution was kept at 37 °C for 1.5 h with continuous stirring. After the incubation was completed, the solution was centrifuged at 8,000 rpm for 30 min.

The pellet was dissolved in 0.5 M NaCl (in a volume that is 2-2.5-fold lower than the initial supernatant volume) at 37 °C with stirring; after the pellet was completely dissolved, an equal volume of isopropanol (1:1) was added, and the mixture was left at -20 °C overnight. The formed hyaluronic acid pellet was collected by centrifugation at 8,000 rpm for 30 min and collected for further use.

The hyaluronic acid pellet was diluted in distilled water until completely dissolved. The resulting viscous yellowish liquid was clarified on the activated carbon column. If the liquid is cloudy, it was passed through 0.45 µm filter. The elution was carried out with distilled water; the eluate should be colorless or of light cream color. If the color was more intense, the preparation was applied on the activated carbon one more time.

To the HA sample, which was previously acidified with 100% acetic acid to pH 3.5, 10% bentonite suspension comprising 0.5% Na₂CO₃ was added to the concentration of 0.1%. The solution was incubated for 2 hours with stirring at 150 rpm. After incubation, the solution was centrifuged at 8,000 rpm or filtered through 0.45 µm filter.

Ultrafiltration through Pellicon 2-300 kDa membrane. After passing through 0.45 µm filter, the hyaluronic acid preparation was filtered to remove any remaining cetylpyridinium and salt against 10-20-fold excess volume of distilled water. The conductivity of the HA preparation should not exceed 2 mS/cm. The final HA preparation was left for 1 to 2 days at 40°C. If the preparation becomes cloudy after a day of storage, it was passed through the 0.45 µm or 0.22 µm filter.

### Example 7. Levels of hyaluronic acid synthesis upon cultivation of the obtained genetic constructs.

The produced genetic constructs comprising, in their chromosome, the fusion of the heterologous *hasA* gene from *S. equisimilis* with *tuaD* gene of *B. subtilis* under control of the hybrid tandem P_{rpsFgsiB} promoter, and further comprising *gtaB* gene under control of the hybrid tandem P_{rpsFgsiB} promoter control and/or gcaD-prs operon under control of the hybrid tandem P_{rpsFgsiB} promoter were tested for ability to synthesize hyaluronic acid during cultivation in a laboratory scale fermenter (3 L) using the fermentation medium described in Example 7.

The results are shown in Table 1.

**Table 1. Kinetics of hyaluronic acid accumulation in culture broth of the obtained genetic constructs producing hyaluronic acid**

| **Producing strain** | **Genotype** | **Fermentation time (hours)** | |
|---|---|---|---|
| | | **24** | **48** |
| | | **Hyaluronic acid accumulation (g/L)** | |
| ***B. subtilis* 168 (original strain)** | **Wild type** | **<0.01** | **<0.01** |
| **AM2640** | *amyE*:: P*_{rpsF-gsiB}-hasA-tuaD* | **1.5** | **1.8** |
| **AM2681** | *amyE*::P*_{rpsF-gsiB}-hasA-tuaD* | **1.65** | **2.21** |
| | P*_{rpsF-gsiB}-gtaB* | | |
| **AM2686** | *amyE*::P*_{rpsF-gsiB}-hasA-tuaD* | **1.96** | **2.83** |
| | P*_{rpsF-gsiB}-gcaD-prs* | | |
| **AM2694** | *amyE*::*P_{rpsF-gsiB}-hasA-tuaD* | **2.35** | **3.21** |
| | P*_{rpsF-gsiB}-gtaB* P*_{rpsF-gsiB}-gcaD-prs* | | |

As seen from Table 1, each of the obtained genetic constructs exhibited a higher level of the target product accumulation compared to the original *B. subtilis* strain 168. The AM2694 genetic construct comprising *gtaB* gene and *gcaD-prs* operon under control of the hybrid tandem P_{rpsFgsiB} promoter in its chromosome in addition to amyE::PrpsF-gsiB-hasA-tuaD, showed the highest hyaluronic acid accumulation. This genetic construct yielded 3.21 g hyaluronic acid per liter.

## Claims

1. A transformed cell of a bacterium of the genus *Bacillus* producing hyaluronic acid and comprising in its chromosome a fusion of heterologous *hasA* gene from *S. equisimilis* with *tuaD* gene of *B. subtilis* under control of a hybrid tandem PrpsF-gsiB promoter, wherein the cell optionally comprises in its chromosome *gtaB* gene under control of the hybrid tandem PrpsF-gsiB promoter, and/or *gcaD-prs* operon under control of the hybrid tandem PrpsF-gsiB promoter.

2. The transformed cell of claim 1, wherein the cell comprises in its chromosome *gtaB* gene under control of the hybrid tandem PrpsF-gsiB promoter.

3. The transformed cell of claim 1, wherein the cell comprises in its chromosome gcaD-prs operon under control of the hybrid tandem PrpsF-gsiB promoter.

4. The transformed cell of claim 1, wherein the cell comprises in its chromosome gtaB gene under control of the hybrid tandem PrpsF-gsiB promoter, and gcaD-prs operon under control of the hybrid tandem PrpsF-gsiB promoter.

5. The transformed cell according to any one of claims 1 to 4, wherein the bacterium of the genus *Bacillus* is *B. subtilis.*

6. A method for producing hyaluronic acid comprising:
(a) culturing the transformed cell according to any one of claims 1 to 5 in a nutrient medium suitable for hyaluronic acid production, and
(b) isolating hyaluronic acid from the culture broth.

7. The method according to claim 6, wherein the liquid nutrient medium of the following composition is used (wt. %):
| |
|---|
| Sucrose - 8.0; |
| Maltose - 4.0; |
| Yeast extract - 0.5; |
| Urea - 0.6; |
| KH₂PO₄ - 0.6; |
| K₂HPO₄ - 1.4; |
| (NH₄)₂SO₄ - 0.3; |
| Sodium citrate - 0.2; |
| Casamino acids - 0.04; |
| MgSO₄ - 0.3; |
| Water - QS. |


SEQUENCE LISTING

<110>  OBSHCHESTVO S OGRANICHENNOI OTVETSTVENNOSTYU "CENTR
       TRANSFERA BIOTECHNOLOGIY OKA-BIOTEH"

<120>  BACTERIA OF THE GENUS  BACILLUS PRODUCING HYALURONIC ACID

<130>

<140>  PCT/RU2020/000311
<141>  2020-06-26

<150>  RU 2019123612
<151>  2019-07-26

<160>  23

<170>  PatentIn version 3.5

<210>  1
<211>  210
<212>  DNA
<213>  Artificial Sequence

<220>
<223>  Hybrid tandem promoter PrpsF-gsiB

<400>  1
cttgaaggga aagaatatgt ggtccaagac ggagatgtta ttcatttccg atttaatgta      60

taggatgcag ttgtaaaggg acaagagctt tggtataata taaaattgtg agtaatagaa     120

ttattgctcc ttgcccatta tggggtttgt ttaaaagaat tgtgagcggg aatacaacaa     180

caacaccaat taaaggagga attcaaaatg                                      210


<210>  2
<211>  18
<212>  DNA
<213>  Artificial Sequence

<220>
<223>  Primer 1

<400>  2
taagggtaac tattgccg                                                    18


<210>  3
<211>  20
<212>  DNA
<213>  Artificial Sequence

<220>
<223>  Primer 2

<400>  3
taagttgggt aacgccaggg                                                  20


<210>  4
<211>  37

<212>  DNA
<213>  Artificial Sequence

<220>
<223>  Primer 3

<400>  4
cgcggatcct aaatgagaac attaaaaaac ctcataa                                37


<210>  5
<211>  46
<212>  DNA
<213>  Artificial Sequence

<220>
<223>  Primer 4

<400>  5
ttcattttga attcctcctt tttataataa ttttttacgt gttccc                     46


<210>  6
<211>  46
<212>  DNA
<213>  Artificial Sequence

<220>
<223>  Primer 5

<400>  6
tataaaaagg aggaattcaa aatgaaaaaa atagctgtca ttggaa                      46


<210>  7
<211>  27
<212>  DNA
<213>  Artificial Sequence

<220>
<223>  Primer 6

<400>  7
cccgcggccg ccgagcctag cagccgg                                          27


<210>  8
<211>  25
<212>  DNA
<213>  Artificial Sequence

<220>
<223>  Primer 7

<400>  8
cgcctgcagg agctaaagag gcagg                                            25


<210>  9
<211>  30
<212>  DNA
<213>  Artificial Sequence

<220>
<223>  Primer 8

<400>  9
cgcaagcttc ctcctttaat tggtgttggt                                        30


<210>  10
<211>  32
<212>  DNA
<213>  Artificial Sequence

<220>
<223>  Primer 9

<400>  10
cgcccgcgga tatctttaaa caactcatcc aa                                     32


<210>  11
<211>  33
<212>  DNA
<213>  Artificial Sequence

<220>
<223>  Primer 10

<400>  11
cgcctgcagc tttatcctta atattaaaac tta                                    33


<210>  12
<211>  33
<212>  DNA
<213>  Artificial Sequence

<220>
<223>  Primer 11

<400>  12
cgcaagcttt aaatgaaaaa agtacgtaaa gcc                                    33


<210>  13
<211>  26
<212>  DNA
<213>  Artificial Sequence

<220>
<223>  Primer 12

<400>  13
cgcggtacca agcagtaccg caaacg                                            26


<210>  14
<211>  20
<212>  DNA
<213>  Artificial Sequence

<220>
<223>  Primer 13

<400>  14
cttcacttgt cagcgggtca                                                    20


<210>  15
<211>  29
<212>  DNA
<213>  Artificial Sequence

<220>
<223>  Primer 14

<400>  15
cgcccgcggt gaactactgt ggaagttac                                          29


<210>  16
<211>  31
<212>  DNA
<213>  Artificial Sequence

<220>
<223>  Primer 15

<400>  16
cgcctgcagt gcaagaattt tctattgagt g                                       31


<210>  17
<211>  29
<212>  DNA
<213>  Artificial Sequence

<220>
<223>  Primer 16

<400>  17
cgcatcgatg gataagcggt ttgcagttg                                          29


<210>  18
<211>  29
<212>  DNA
<213>  Artificial Sequence

<220>
<223>  Primer 17

<400>  18
cgcggtaccg cagttaaaat cgtagcttt                                          29


<210>  19
<211>  22
<212>  DNA
<213>  Artificial Sequence

<220>
<223>  Primer 18

<400>  19
attacgtacc ggtgttgatc tc                                                 22

<210>  20
<211>  28
<212>  DNA
<213>  Artificial Sequence

<220>
<223>  Primer 19

<400>  20
cgcccgcggc aactttatgc ccatgcaa                                          28


<210>  21
<211>  28
<212>  DNA
<213>  Artificial Sequence

<220>
<223>  Primer 20

<400>  21
cgcctgcagt gcggtagtaa aggttaat                                          28


<210>  22
<211>  28
<212>  DNA
<213>  Artificial Sequence

<220>
<223>  Primer 21

<400>  22
cgcctgcagc atccttgaac aaggacaa                                          28


<210>  23
<211>  25
<212>  DNA
<213>  Artificial Sequence

<220>
<223>  Primer 22

<400>  23
cgcctcgaga cggcatggcg ccatt                                             25